# EUROPEAN PATENT APPLICATION

(11) **EP 4 410 271 A1**
(43) Date of publication of application: **07.08.2024**
(21) Application number: 21930600.8
(22) Date of filing: 05.11.2021
(51) Int. Cl.: A61K 8/49, A61K 8/04, A61K 8/02, A61K 8/39, A61K 8/92, A61Q 19/08

(54) **OIL-DISPERSED SOLID COSMETIC COMPOSITION FOR IMPROVING SKIN ELASTICITY INCLUDING CAFFEINE AND PREPARATION METHOD THEREFOR**

(30) Priority: 27.09.2021 KR 20210127447
(71) Applicant: Cosmax, Inc., Hwaseong-si, Gyeonggi-do 18622 (KR)
(72) Inventor: KIM, Seo Yeon, Seoul 06787 (KR); LIM, Hyeon Joo, Gyeonggi-do 13523 (KR); JI, Jin Goo, Gyeonggi-do 16363 (KR); PARK, Myeong Sam, Seoul 04987 (KR)
(74) Representative: Plougmann Vingtoft a/s
(86) International application number: PCT/KR2021/015964
(87) International publication number: WO 2023/048328

(57) **Abstract**

The present invention relates to an oil-dispersion solid cosmetic composition and a method for preparing the same, wherein the cosmetic composition can maintain a stabilized formulation while containing caffeine, and thus can be effectively used in the manufacture of a product for improving skin elasticity.

## Description

### Technical Field

This application claims priority to Korean Patent Application No. 10-2021-0127447 filed in the Korean Intellectual Property Office on 27 September 2021, the disclosure of which is incorporated herein by reference.

The present disclosure relates to an oil-dispersion solid cosmetic composition containing caffeine for improving skin elasticity and a preparation method therefor.

### Background Art

Caffeine is well known to quickly deliver moisture and nutrients to cells by promoting blood circulation. Cellulite is caused by the accumulation of fat due to poor blood and lymph circulation, and caffeine is effective in the management of cellulite in thighs and the buttocks since caffeine converts the fat cells into energy and widens blood vessels to help waste to escape easily.

However, oil-dispersion solid cosmetic materials are not easy to mix with a water-soluble raw material, so that caffeine, a water-soluble ingredient, is highly likely to have a negative effect on formulation stability, and thus caffeine is difficult to apply as it is to the oil-dispersion cosmetic materials and is not easy to uniformly disperse and distribute in products.

### Disclosure of Invention

### Technical Problem

The present inventors prepared a stick-type stabilized oil-dispersion solid cosmetic composition containing caffeine and confirmed that the cosmetic composition had excellent formulation stability as well as superior skin elasticity improvement effects.

Accordingly, an aspect of the present disclosure is to provide an oil-dispersion solid cosmetic composition containing caffeine.

Another aspect of the present disclosure is to provide a method for preparing an oil-dispersion solid cosmetic composition, the method including mixing caffeine and an oil to prepare a mixture.

Still another aspect of the present disclosure is directed to use of an oil-dispersion solid cosmetic composition containing caffeine for improving skin elasticity.

### Solution to Problem

The present disclosure relates to an oil-dispersion solid cosmetic composition containing caffeine and a preparation method therefor, and the cosmetic composition of the present disclosure shows a stabilized formulation in which caffeine is uniformly dispersed, and exerts an effect of improving skin elasticity.

The present inventors prepared a stick-type oil-dispersion solid cosmetic composition having a stabilized formulation while containing caffeine, by adjusting the contents of caffeine and an oil contained in the entire cosmetic composition to search for an optimal condition.

Hereinafter, the present disclosure will be described in more detail.

In accordance with an aspect of the present disclosure, there is provided an oil-dispersion solid cosmetic composition containing caffeine.

In the present disclosure, the cosmetic composition may contain caffeine in a content of 0.01 to 5.00 wt%, preferably, 0.01 to 4.00 wt%, 0.01 to 3.00 wt%, 0.01 to 2.00 wt%, 0.01 to 1.00 wt%, 0.10 to 5.00 wt%, 0.10 to 4.00 wt%, 0.10 to 3.00 wt%, 0.10 to 2.00 wt%, 0.10 to 1.00 wt%, 0.50 to 5.00 wt%, 0.50 to 4.00 wt%, 0.50 to 3.00 wt%, or 0.50 to 2.00 wt%, and for example, 0.50 to 1.00 wt%, but is not limited thereto.

A too large content of caffeine contained in the cosmetic composition may degrade formulation stability, which may cause a problem in that a stick-type formulation as a solid agent, and may degrade spreadability. Conversely, a too small content of caffeine may not sufficiently exert an effect of improving skin elasticity and may also cause a problem in which the stickiness induced by an oil ingredient used together cannot be reduced.

In the present disclosure, the cosmetic composition may further contain an oil in a content of 20.00 to 75.00 wt%, preferably, 25.00 to 75.00 wt%, 30.00 to 75.00 wt%, 35.00 to 75.00 wt%, 40.00 to 75.00 wt%, 45.00 to 75.00 wt%, 50.00 to 75.00 wt%, 55.00 to 75.00 wt%, 60.00 to 75.00 wt%, or 65.00 to 75.00 wt%, and for example, 70.00 to 75.00 wt%, but is not limited thereto.

The oil may be a vegetable oil, an animal oil, a mineral oil, a synthetic oil, or a mixture thereof, but is not particularly limited thereto, and any oil that can be used in cosmetic products can be used.

The oil may be preferably at least one selected from the group consisting of polyglyceryl-2 triisostearate, sunflower seed oil, coconut oil, sweet almond oil, apple seed oil, octyldodecanol, hydrogenated castor oil, meadowfoam seed oil, triethylhexanoin, diisostearyl malate, olive oil, castor oil, palm oil, avocado oil, almond oil, rosehip oil, macadamia nut oil, mink oil, liquid paraffin, Vaseline, isopropyl myristate, silicon oil, Jojoba oil, green tea oil, grape seed oil, argan oil, and hydrogenated polydecene, but is not limited thereto.

The oil may be more preferably at least one selected from the group consisting of polyglyceryl-2 triisostearate, sunflower seed oil, coconut oil, sweet almond oil, apple seed oil, octyldodecanol, hydrogenated castor oil, meadowfoam seed oil, triethylhexanoin, and diisostearyl malate, and may be for example at least one selected from the group consisting of polyglyceryl-2 triisostearate, sunflower seed oil, coconut oil, sweet almond oil, apple seed oil, octyldodecanol, and hydrogenated castor oil, but is not limited thereto.

In the present disclosure, the cosmetic composition may further contain a butter in a content of 2.00 to 10.00 wt%, and preferably 5.00 to 8.00 wt%.

The butter may be a vegetable butter, and preferably, at least one selected from the group consisting of shea butter, mango seed butter, cocoa seed butter, cupuacu seed butter, coconut seed butter, *Shorea robusta* seed butter, and *Garcinia indica* seed butter, and for example, shea butter, but is not limited thereto.

In the present disclosure, the cosmetic composition may further contain a wax in a content of 10.00 to 30.00 wt%, and preferably 15.00 to 25.00 wt%.

The wax may be at least one selected from the group consisting of candelilla wax and microcrystalline wax.

In the present disclosure, the cosmetic composition may further contain at least one selected from the group consisting of cyclodextrin, tocopherol, and a preservative.

In an embodiment of the present disclosure, an oil-dispersion solid cosmetic composition containing 0.01 to 5.00 wt% of caffeine, 20.00 to 75.00 wt% of an oil, 10.00 to 30.00 wt% of a wax can be prepared.

In a specific embodiment of the present disclosure, an oil-dispersion solid cosmetic composition containing 0.01 wt% of caffeine, 70.00 to 75.00 wt% of an oil, 15.00 to 25.00 wt% of a wax can be prepared.

In another aspect of the present disclosure, there is provided a method for preparing an oil-dispersion solid cosmetic composition, the method including mixing caffeine and an oil to prepare a mixture.

In the present disclosure, the mixing step may be performed at 60 to 100°C, and preferably 60 to 95°C, 60 to 90°C, 60 to 85°C, 65 to 100°C, 65 to 95°C, 65 to 90°C, 65 to 85°C, 70 to 100°C, 70 to 95°C, or 70 to 90°C, and for example, 70 to 85°C, but is not limited thereto.

The mixture may contain caffeine in a content of 0.01 to 5.00 wt%, preferably, 0.01 to 4.00 wt%, 0.01 to 3.00 wt%, 0.01 to 2.00 wt%, 0.01 to 1.00 wt%, 0.10 to 5.00 wt%, 0.10 to 4.00 wt%, 0.10 to 3.00 wt%, 0.10 to 2.00 wt%, 0.10 to 1.00 wt%, 0.50 to 5.00 wt%, 0.50 to 4.00 wt%, 0.50 to 3.00 wt%, or 0.50 to 2.00 wt%, and for example, 0.50 to 1.00 wt%, but is not limited thereto.

A too high content of caffeine contained in the mixture may degrade formulation stability, which may cause a problem in that a stick formulation cannot be formed, and may degrade spreadability. Conversely, a too small content of caffeine may not sufficiently exert an effect of improving skin elasticity and may also cause a problem in which the stickiness due to an oil component used together cannot be reduced.

The mixture may further contain an oil in a content of 20.00 to 75.00 wt%, preferably, 25.00 to 75.00 wt%, 30.00 to 75.00 wt%, 35.00 to 75.00 wt%, 40.00 to 75.00 wt%, 45.00 to 75.00 wt%, 50.00 to 75.00 wt%, 55.00 to 75.00 wt%, 60.00 to 75.00 wt%, or 65.00 to 75.00 wt%, and for example, 70.00 to 75.00 wt%, but is not limited thereto.

The oil may be a vegetable oil, an animal oil, a mineral oil, a synthetic oil, or a mixture thereof, but is not particularly limited thereto, and any oil that can be used in cosmetic products can be used.

The oil may be at least one selected from the group consisting of polyglyceryl-2 triisostearate, sunflower seed oil, coconut oil, sweet almond oil, apple seed oil, octyldodecanol, hydrogenated castor oil, meadowfoam seed oil, triethylhexanoin, diisostearyl malate, olive oil, castor oil, palm oil, avocado oil, almond oil, rosehip oil, macadamia nut oil, mink oil, liquid paraffin, Vaseline, isopropyl myristate, silicon oil, Jojoba oil, green tea oil, grape seed oil, argan oil, and hydrogenated polydecene, but is not limited thereto.

The oil may be more preferably at least one selected from the group consisting of polyglyceryl-2 triisostearate, sunflower seed oil, coconut oil, sweet almond oil, apple seed oil, octyldodecanol, hydrogenated castor oil, meadowfoam seed oil, triethylhexanoin, and diisostearyl malate, and may be for example at least one selected from the group consisting of polyglyceryl-2 triisostearate, sunflower seed oil, coconut oil, sweet almond oil, apple seed oil, octyldodecanol, and hydrogenated castor oil, but is not limited thereto.

In the present disclosure, the mixture may further contain a butter in a content of 2.00 to 10.00 wt%, and preferably 5.00 to 8.00 wt%.

The butter may be a vegetable butter, and preferably, at least one selected from the group consisting of shea butter, mango seed butter, cocoa seed butter, cupuacu seed butter, coconut seed butter, *Shorea robusta* seed butter, and *Garcinia indica* seed butter, and for example, shea butter, but is not limited thereto.

In the present disclosure, the mixture may further contain a wax in a content of 10.00 to 30.00 wt%, and preferably 15 to 25 wt%.

The wax may be at least one selected from the group consisting of candelilla wax and microcrystalline wax.

In the present disclosure, the mixture may further contain at least one selected from the group consisting of cyclodextrin, tocopherol, and a preservative.

In the present disclosure, the method for preparing an oil-dispersion solid cosmetic composition may further include a molding step of placing the mixture in a container, followed by cooling, thereby molding the mixture in a desired shape. The molding may be performed for a stick shape, but is not limited thereto.

### Advantageous Effects of Invention

The present disclosure is directed to an oil-dispersion solid cosmetic composition and a method for preparing the same, wherein the cosmetic composition can maintain a stabilized formulation while containing caffeine, and thus can be effectively used in the manufacture of a product for improving skin elasticity.

### Brief Description of Drawings

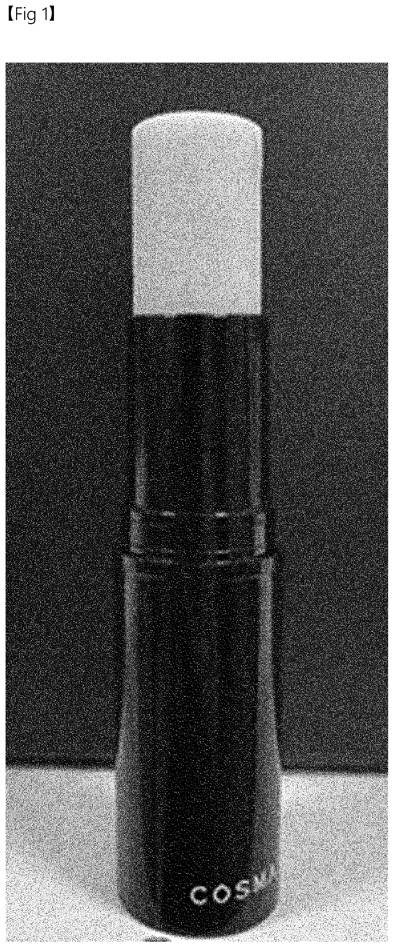

FIG. 1 is an image showing a formulation of a cosmetic composition prepared according to an embodiment of the present disclosure.

### Best Mode for Carrying out the Invention

The present disclosure is directed to an oil-dispersion solid cosmetic composition containing caffeine.

### Mode for Carrying out the Invention

Hereinafter, the present disclosure will be described in more detail by the following examples. However, these exemplary embodiments are used only for illustration, and the scope of the present disclosure is not limited by these exemplary embodiments.

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Preparation Example 1: Preparation of cosmetic composition

Oil-dispersion solid cosmetic compositions containing caffeine were prepared according to the compositions (unit: wt%) in Table 1 below.

**TABLE 1**

| Classifica tion | Ingredient | Example 1 | Comparativ e Example 1 | Comparativ e Example 2 | Comparativ e Example 3 |
|---|---|---|---|---|---|
| Caffeine | Caffeine | 1.0 | 0.001 | 6.0 | 10.0 |
| Oil | Polyglycer yl-2 triisostea rate, | 30.0 | 30.0 | 30.0 | 30.0 |
| | Sunflower seed oil | 14.8 | 15.799 | 9.8 | 5.8 |
| | Coconut oil | 9.9 | 9.9 | 9.9 | 9.9 |
| | Sweet almond oil | 5.0 | 5.0 | 5.0 | 5.0 |
| | Apple seed oil | 5.0 | 5.0 | 5.0 | 5.0 |
| | Octyldodec anol | 3.0 | 3.0 | 3.0 | 3.0 |
| | Hydrogenat ed castor oil | 3.5 | 3.5 | 3.5 | 3.5 |
| Vegetable butter | Shea butter | 5.0 | 5.0 | 5.0 | 5.0 |
| Wax | Candelilla wax | 15.0 | 15.0 | 15.0 | 15.0 |
| | Microcryst alline wax | 5.0 | 5.0 | 5.0 | 5.0 |
| - | Cyclodextr in | 2.5 | 2.5 | 2.5 | 2.5 |
| - | Tocopherol | 0.1 | 0.1 | 0.1 | 0.1 |
| Preservati ve | Glyceryl caprylate | 0.2 | 0.2 | 0.2 | 0.2 |

All the raw materials were weighed in beakers according to each ingredient and content thereof shown in Table 1, and then uniformly mixed at 25 rpm for 5 minutes by Agi-Mixer while warmed at 75-85°C. Then, the mixtures were defoamed, poured into stick containers, and cooled, thereby preparing stick type cosmetic compositions.

### Test Example 1: Formulation stability

The cosmetic compositions prepared in Preparative Example 1 were examined for formulation stability.

Specifically, each cosmetic composition was placed under conditions of 4°C, room temperature, 37°C, 45°C, and light for 1 day, 5 days, 1 week, 2 weeks, and 4 weeks, and then evaluated for formulation stability. The results are shown in Table 2 below. (Evaluation criteria: ⊚ Stable, △ Slight unstable, X Unstable)

**TABLE 2**

| | | Example 1 | Comparative Example 1 | Comparative Example 2 | Comparative Example 3 |
|---|---|---|---|---|---|
| 1 Days | 4°C | ⊚ | ⊚ | ⊚ | X |
| | Room temperature | ⊚ | ⊚ | ⊚ | X |
| | 37°C | ⊚ | ⊚ | ⊚ | X |
| | 45°C | ⊚ | ⊚ | ⊚ | X |
| | Light | ⊚ | ⊚ | ⊚ | X |
| 5 Days | 4°C | ⊚ | ⊚ | ⊚ | X |
| | Room temperature | ⊚ | ⊚ | ⊚ | X |
| | 37°C | ⊚ | ⊚ | ⊚ | X |
| | 45°C | ⊚ | ⊚ | ⊚ | X |
| | Light | ⊚ | ⊚ | ⊚ | X |
| 1 Week | 4°C | ⊚ | ⊚ | ⊚ | X |
| | Room temperature | ⊚ | ⊚ | ⊚ | X |
| | 37°C | ⊚ | ⊚ | ⊚ | X |
| | 45°C | ⊚ | ⊚ | Δ | X |
| | Light | ⊚ | ⊚ | ⊚ | X |
| 2 Weeks | 4°C | ⊚ | ⊚ | ⊚ | X |
| | Room temperature | ⊚ | ⊚ | ⊚ | X |
| | 37°C | ⊚ | ⊚ | Δ | X |
| | 45°C | ⊚ | ⊚ | Δ | X |
| | Light | ⊚ | ⊚ | Δ | X |
| 4 Weeks | 4°C | ⊚ | ⊚ | ⊚ | X |
| | Room temperature | ⊚ | ⊚ | ⊚ | X |
| | 37°C | ⊚ | ⊚ | Δ | X |
| | 45°C | ⊚ | ⊚ | Δ | X |
| | Light | ⊚ | ⊚ | Δ | X |

As can confirmed in Table 2, Example 1 and Comparative Example 1 showed a favorable stable formulation even 4 weeks after preparation. However, Comparative Example 2 was observed to have an unstable formulation from 5 days after preparation, and Comparative Example 3 was evaluated as having a formulation stability that was too low to form a stick.

As can be confirmed from FIG. 1, caffeine in the cosmetic composition according to Example 1 was uniformly dispersed and thus stabilized without precipitation, indicating that a stabilized oil-dispersion cosmetic composition containing caffeine was prepared.

### Test Example 2: Skin elasticity improving effect

The cosmetic compositions prepared in Preparative Example 1 were examined for a skin elasticity improving effect.

Specifically, 15 female professional subjects aged 20 to 50 were divided into two groups, and Example 1 and Comparative Examples 1 and 2 were applied at an appropriate amount onto the skin of the abdomen area in the morning and evening for 4 weeks. Comparative Example 3 was excluded from evaluation since a stick was not formed due to unstable formulation stability. The skin elasticity improving effect was examined using Cutometer MPA580 (Courage and Khazaka Electronic GmbH, Germany).

**TABLE 3**

| | Example 1 | Comparative Example 1 | Comparative Example 2 |
|---|---|---|---|
| Skin elasticity before use | 0.798 | 0.782 | 0.778 |
| Skin elasticity 4 weeks after use | 0.872 | 0.791 | 0.845 |
| Skin elasticity improvement (%) | 109.27 | 101.15 | 108.61 |

As can be confirmed in Table 3 above, Example 1 and Comparative Example 2 were effective in skin elasticity improvement. However, Comparative Example 1 was not effective in skin elasticity improvement.

### Test Example 3: Spreadability and stickiness evaluation

User test results for spreadability and stickiness of the cosmetic compositions prepared in Preparation Example 1 were investigated.

Specifically, 30 female professional subjects aged 25 to 45 were divided into two groups, and the amounts of Example 1 and Comparative Examples 1 and 2 were appropriately adjusted for each group and applied onto the skin of the face and abdomen, and then evaluated for spreadability and stickiness according to the following evaluation criteria. Comparative Example 3 was excluded from the sensory evaluation since a stick was not formed due to unstable formulation stability.

In the evaluation criteria, the spreadability was evaluated by checking whether the prepared cosmetic compositions were smoothly spread on the face and abdomen. The stickiness was evaluated by checking the degree of stickiness felt in the hand when the prepared compositions were applied to the face and abdomen and then allowed to be absorbed thereinto.

The evaluation of each item was carried out according to a 5-point scale including very high (5 points), high (4 points), intermediate (3 points), low (2 points), and very low (1 point). As for spreadability, a higher score indicates better spreadability, and as for the stickiness, a lower score indicates being less sticky. The evaluation results are shown in Table 4.

**TABLE 4**

| Classification | Example 1 | Comparative Example 1 | Comparative Example 2 |
|---|---|---|---|
| Spreadability | 4.2 | 4.1 | 1.8 |
| Stickiness | 1.8 | 4.8 | 1.5 |

As can be confirmed from Table 4, Example 1 showed higher evaluations in spreadability and stickiness compared with Comparative Examples 1 and 2. However, Comparative Example 1 showed excellent spreadability but was inconvenient to use on the skin due to high stickiness. Comparative Example 2 was excellent due to low stickiness but was difficult to apply at an appropriate amount on the skin due to very low spreadability.

### Industrial Applicability

The present disclosure relates to an oil-dispersion solid cosmetic composition containing caffeine for improving skin elasticity and a preparation method therefor.

## Claims

1. An oil-dispersion solid cosmetic composition comprising caffeine.

2. The cosmetic composition of claim 1, wherein the cosmetic composition comprises 0.01 to 5.00 wt% of caffeine.

3. The cosmetic composition of claim 1, wherein the cosmetic composition further comprises 20.00 to 75.00 wt% of an oil.

4. The cosmetic composition of claim 3, wherein the oil is at least one selected from the group consisting of polyglyceryl-2 triisostearate, sunflower seed oil, coconut oil, sweet almond oil, apple seed oil, octyldodecanol, hydrogenated castor oil, meadowfoam seed oil, triethylhexanoin, and diisostearyl malate.

5. The cosmetic composition of claim 1, wherein the cosmetic composition further comprises a butter or a wax.

6. A method for preparing an oil-dispersion solid cosmetic composition, the method comprising mixing caffeine and an oil to prepare a mixture.

7. The method of claim 6, wherein the cosmetic composition contains 0.01 to 5.00 wt% of caffeine.

8. The method of claim 6, wherein the cosmetic composition comprises 20.00 to 75.00 wt% of the oil.

9. The method of claim 6, wherein the oil is at least one selected from the group consisting of polyglyceryl-2 triisostearate, sunflower seed oil, coconut oil, sweet almond oil, apple seed oil, octyldodecanol, hydrogenated castor oil, meadowfoam seed oil, triethylhexanoin, and diisostearyl malate.

10. The method of claim 6, wherein the cosmetic composition further comprises a butter or a wax.
